# EUROPÄISCHE PATENTSCHRIFT

(11) **EP 0 946 514 B1**
(45) Veröffentlichungstag und Bekanntmachung des Hinweises auf die Patenterteilung: **02.05.2003**
(21) Anmeldenummer: 97953736.2
(22) Anmeldetag: 04.12.1997
(51) Int. Cl.: C07D 231/20, C07D 231/22

(54) **VERFAHREN ZUR HERSTELLUNG VON N-SUBSTITUIERTEN 3-HYDROXYPYRAZOLEN**
METHOD FOR PRODUCING N-SUBSTITUTED 3-HYDROXYPYRAZOLES
PROCEDE POUR LA PREPARATION DE 3-HYDROXYPYRAZOLS N-SUBSTITUES

(30) Priorität: 17.12.1996 DE 19652516
(43) Veröffentlichungstag der Anmeldung: 06.10.1999
(73) Patentinhaber: BASF AKTIENGESELLSCHAFT, 67056 Ludwigshafen (DE)
(72) Erfinder: VOGELBACHER, Uwe, Josef, D-67071 Ludwigshafen (DE); KEIL, Michael, D-67251 Freinsheim (DE); KLINTZ, Ralf, D-67269 Grünstadt (DE); WAHL, Joseph, D-67105 Schifferstadt (DE); WINGERT, Horst, D-68159 Mannheim (DE); KÖNIG, Hartmann, D-69115 Heidelberg (DE); RACK, Michael, D-69123 Heidelberg (DE); GÖTZ, Roland, D-91541 Rothenburg (DE); TELES, Joaquim, Henrique, D-67059 Ludwigshafen (DE)
(74) Vertreter: Fitzner, Uwe, Dr.
(86) Internationale Anmeldenummer: EP9706780
(87) Internationale Veröffentlichungsnummer: WO98027062

(56) Entgegenhaltungen:
- EP-A- 0 162 247
- WO-A-97/03969
- JOURNAL OF MEDICINAL CHEMISTRY, Bd. 34, Nr. 5, Mai 1991, Seiten 1560-1570, XP002061838 in der Anmeldung erwähnt

## Beschreibung

Die vorliegende Erfindung betrifft ein Verfahren zur Herstellung von N-substituierten 3-Hydroxypyrazolen der Formel I 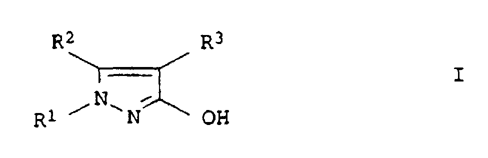 in der
- R¹, R² und R³: die in Anspruch 1 angegebene Bedentung haben,
durch Oxidation eines Pyrazolidin-3-ons der Formel II.

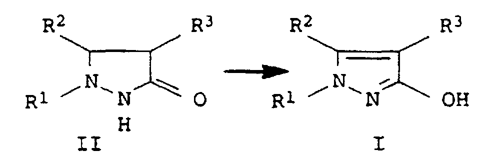

Aus der Literatur ist bekannt, daß man N-substituierte 3-Hydroxypyrazole durch Oxidation der entsprechenden Pyrazolidinone erhält [J. Gen. Chem. USSR, Engl. Trans. 31, 1770 (1961); Chem. Heterocycl. Comp. 5, 527 (1969); J. Prakt. Chem. 313, 115 (1971); J. Prakt. Chem. 318, 253 (1976); J. Med. Chem. 34, 1560 (1991); J. Prakt. Chem. 313, 1118 (1971); DE-A 34 15 385; PCT/EP 96/02,891].

Als Oxidationsmittel finden dabei
- elementarer Schwefel [J. Gen. Chem. USSR, Engl. Trans. 31, 1770 (1961)],
- elementare Halogene [Chem. Heterocycl. Comp. 5, 527 (1969); J. Prakt. Chem. 318, 253 (1976); J. Prakt. Chem. 313, 1118 (1971)],
- Peroxide [J. Med. Chem. 34, 1560 (1991); DE-A 34 15 385] und
- Luftsauerstoff [J. Prakt. Chem. 313, 115 (1971); J. Prakt. Chem. 313, 1118 (1971); PCT/EP 96/02,891]
Verwendung.

Im Hinblick auf eine technische Herstellung der 3-Hydroxypyrazole hat die Oxidation mit elementarem Schwefel den Nachteil, daß erhebliche Mengen an Reduktionsprodukten des Schwefels gebildet werden, die eine aufwendige Aufarbeitung und Entsorgung erforderlich machen.

Die Verwendung von elementaren Halogenen eignet sich ebenfalls nicht für eine technische Synthese der 3-Hydroxypyrazole, da die Ausbeuten zu wünschen übrig lassen und die Abtrennung der in erheblichem Umfang entstehenden Nebenprodukte aufwendig ist. Außerdem ist die Verwendung großer Mengen an elementarem Halogen als Oxidationsmittel sowohl aus Umweltgründen als auch im Hinblick auf die Kosten nachteilig.

Die bekannten Oxidationsverfahren mit Peroxiden erfordern einerseits aufwendige Reinigungen und bieten andererseits bei der Verwendung teurer Reagenzien nur eine unbefriedigende Ausbeute, so daß sie im Hinblick auf eine technische Synthese nicht in Betracht kommen.

Die Verwendung von Luftsauerstoff als Oxidationsmittel [J. Prakt. Chem. 313, 115 (1971) und J. Prakt. Chem. 313, 1118 (1971)] hat den Nachteil, daß die Umsetzung in stark saurem Medium durchgeführt werden muß. Daraus ergibt sich bei der Aufarbeitung ein erheblicher Verbrauch an Basen und daraus resultierend ein beträchtlicher Salzanfall, der aus ökologischer Sicht unerwünscht ist.

PCT/EP 96/02,891 beschreibt die Oxidation mit Luftsauerstoff in organischer Lösung in Gegenwart von Eisen- und Kupfersalzen. Durch das Oxidationsmittel Luft entstehen dabei allerdings explosionsfähige Luft/Lösungsmitteldampf-Gemische, die aus Gründen der Sicherheit bedenklich sind und hohe Anforderungen an die Sicherheitstechnik stellen.

Der vorliegenden Erfindung lag ein wirtschaftliches und technisch sicheres und einfaches Verfahren zur Herstellung von 3-Hydroxypyrazolen als Aufgabe zugrunde.

Demgemäß wurde ein Verfahren zur Herstellung von N-substituierten 3-Hydroxypyrazolen der Formel I 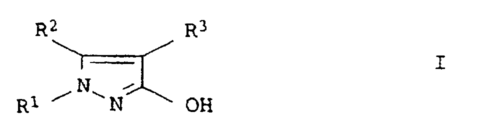 in der
- R¹: für C₁-C₁₀-Alkyl, C₂-C₁₀-Alkenyl, C₂-C₁₀-Alkinyl, C₃-C₁₀-Cycloalkyl,
Aryl ausgewählt aus Phenyl und Naphthyl; Heteroaryl ausgewählt aus Furyl, Thienyl, Pyrrolyl, Isoxazolyl, Isothiazolyl, Pyrazolyl, Oxazolyl, Imidazolyl, Pyridyl, Pyridazinyl, Pyrimidinyl oder Triazinyl; und
- R², R³: für Wasserstoff, Cyano, Halogen und C₁-C₁₀-Alkyl, C₂-C₁₀-Alkenyl, C₂-C₁₀-Alkinyl, C₃-C₁₀-Cycloalkyl; Aryl ausgewählt aus Phenyl und Naphthyl; Heteroaryl ausgewählt aus Furyl, Thienyl, Pyrrolyl, Isoxazolyl, Isothiazolyl, Pyrazolyl, Oxazolyl, Imidazolyl, Pyridyl, Pyridazinyl, Pyrimidinyl oder Triazinyl; steht,
wobei die Substituenten R¹, R² und R³ unter den Reaktionsbedingungen inert sind,
durch
Oxidation eines Pyrazolidin-3-ons der Formel II 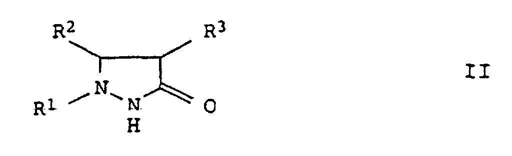 dadurch gekennzeichnet, daß man die Umsetzung der in Wasser löslichen Verbindungen der Formel II in Wasser in Gegenwart einer Base mit Sauerstoff als Oxidationsmittel durchführt.

Bei der Oxidation der Pyrazolidinone II geht man im allgemeinen so vor, daß man eine wässrige basische Lösung von II mit Luft oder reinem Sauerstoff begast.

Als Basen kommen anorganische oder organische in Betracht, deren pKₐ-Wert größer ist als 7.

Das erfindungsgemäße Verfahren erfordert keine vollständige Deprotonierung der Verbindung II. Bei unvollständiger Deprotonierung der Verbindung II liegt der pH-Wert des Reaktionsmediums unter 7. Besonders vorteilhaft wird das Verfahren bei pH-Werten > 7 durchgeführt. Dabei wird die Base mindestens äquimolar zu Verbindung II zugesetzt.

Durch den Basenzusatz wird die Löslichkeit der Pyrazolidione soweit erhöht, daß sie für eine Umsetzung in Wasser zugänglich sind. Um den Kohlenstoffgehalt des Reaktionsabwassers möglichst niedrig zu halten, sind anorganische Basen, wie beispielsweise Hydroxide oder Carbonate der Alkali- oder Erdalkalimetalle, wie z. B. Natriumhydroxid, Kaliumhydroxid, Magnesiumhydroxid, Kaliumcarbonat oder Natriumcarbonat, bevorzugt. Um zu vermeiden, daß bei Eingasen des Sauerstoffs die Lösung an Base verarmt, sind nicht-flüchtige Basen bevorzugt.

Organische Basen sind prinzipiell ebenfalls geeignet. Hierbei sind unter Reaktionsbedingungen nicht-flüchtige Basen nicht nur aus den oben genannten Gründen, sondern auch aus Sicherheitsgründen bevorzugt.

Das Verfahren kann so ausgeführt werden, daß die Oxidation der Reaktionsmischung durch Zusatz katalytischer Mengen eines Metallsalzes beschleunigt wird. Dabei wird in den meisten Fällen auch die Selektivität erhöht.

Als Metallsalze eignen sich insbesondere Salze des Eisens in der zwei- oder dreiwertigen Oxidationsstufe (z.B. Eisen-II-chlorid, Eisen-III-chlorid, Eisen-II-sulfat, Eisen-III-sulfat, Kalium-hexacyanoferrat-II und Kalium-hexacyanoferrat-III), Salze des Kupfers in der ein- oder zweiwertigen Oxidationsstufe (z. B. Kupfer-I-chlorid, Kupfer-II-chlorid, Kupfer-I-sulfat und Kupfer-II-sulfat), Salze des Kobalts in der zwei- oder dreiwertigen Oxidationsstufe (z.B. Kobalt-II-acetat, Kobalt-II-chlorid und Kobalt-III-fluorid), sowie entsprechende Salze von Hauptgruppen- oder Übergangsmetallen. Es können auch mehrere Salze gemeinsam als Mischungen eingesetzt werden.

Die Metallsalze werden im allgemeinen in Mengen von 0,01 mol-% bis 20 mol-%, vorzugsweise 0,3 mol-% bis 10 mol-%, insbesondere 0,5 mol-% bis 5 mol-%, bezogen auf II, eingesetzt.

Eine bevorzugte Ausführungsform des erfindungsgemäßen Verfahrens ist die Oxidation mit reinem Sauerstoff. Dabei kann auf die Metallsalzkatalyse verzichtet werden.

Diese Oxidation erfolgt üblicherweise bei Temperaturen von 0°C bis zum Siedepunkt des Reaktionsgemisches, vorzugsweise 20°C bis 100°C. Bei Durchführung des Verfahrens unter Druck sind auch höhere Temperaturen möglich.

Das Verfahren kann unter 1 bis 200 bar Druck ausgeführt werden. Der Druck kann durch Aufpressen von Luft oder reinem Sauerstoff oder Mischungen daraus aufgebaut werden. Vorteilhaft sind Drucke von 1 bis 50 bar. Insbesondere kommen Drucke von 1 bis 20 bar in Frage.

Die Reaktionsgemische werden in üblicher Weise aufgearbeitet, z.B. durch Ausfällen des Produktes durch Neutralisation der Reaktionslösung, gegebenenfalls Extraktion, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Das erfindungsgemäße Verfahren ist nicht auf bestimmt substituierte Verbindungen beschränkt, sofern die Substituenten unter den Reaktionsbedingungen inert sind. Aliphatische Reste können geradkettig oder verzweigt sein.

Aryl steht für Phenyl oder Naphthyl;
Heteroaryl bedeutet Furyl, Thienyl, Pyrrolyl, Isoxazolyl, Isothiazolyl, Pyrazolyl, Oxazolyl, Imidazolyl, Pyridyl, Pyridazinyl, Pyrimidinyl oder Triazinyl;
Halogen bedeutet Chlor, Fluor, Brom oder Jod.

Die Substituenten können weitere, unter den Reaktionsbedingungen inerte Reste tragen, dabei seien beispielhaft genannt:
Halogen, Cyano, SO₃H, COOH, Alkyl, Alkenyl, Alkinyl, Aryl oder Heteroaryl.

Die nach dem erfindungsgemäßen Verfahren erhältlichen 3-Hydroxypyrazole eignen sich als Zwischenprodukte zur Herstellung von Farbstoffen oder Wirkstoffen auf dem Pharma- oder Pflanzenschutzbereich.

### Vergleichsbeispiele:

1. Oxidation von Pyrazolidinonen mit FeCl₃ [J.prakt.Ch. 313, 1118 (1991)]
   Zum Gemisch aus 14 g (0,071 mol) 1-(4-Chlorphenyl)-pyrazolidin-3-on und 100 ml 1 N HCl wurde bei etwa 25°C eine Lösung von 23 g (0,142 mol) FeCl₃ in 40 ml H₂O zugetropft. Nach Rühren über Nacht gab man portionsweise 24 g NaOH hinzu, erhitzte auf 90°C und saugte heiß ab. Der Niederschlag wurde mit siedendem Wasser gewaschen.
   Nach Ansäuern des Filtrats auf pH 5-6 und anschließender Extraktion mit CHCl₃ erhielt man aus der organischen Phase eine geringe Menge eines dunklen Rückstandes, in dem sich kein Produkt nachweisen ließ.
   Auch aus dem bei der wäßrigen Phase und dem bei der Filtration erhaltenen Festkörper konnte kein Produkt isoliert werden, dessen Reinheit für eine quantitative oder qualitative Charakterisierung ausreichte.
2. Oxidation von Pyrazolidinonen mit CuCl₂ [J.prakt.Ch. 213, 115 (1971)]
   2.1. In ein Gemisch aus 19,6 g (0,1 mol) 1-(4-Chlorphenyl)-pyrazolidin-3-on, 200 ml 1 N HCl und 0,05 g CuCl₂ x 2 H₂O (0,293 mmol) wurde bei 50°C 8 h lang Sauerstoff eingeleitet. Anschließend wurde über Nacht nachgerührt und der entstandene braune Feststoff abgesaugt. Man erhielt 17,7 g eines Gemischs aus Pyrazolinon und Pyrazolidinon im Verhältnis 4:1.
      Berechnete Ausbeute: 73 %.
   2.2. Ein Analogversuch, bei dem man 24 h lang bei 50°C Sauerstoff einleitete, lieferte 17.8 g eines Gemisches, dessen spektroskopische und physikalische Daten identisch mit den unter 2.1. erhaltenen waren. Die während der Reaktion durchgeführte dünnschichtchromatographische Untersuchung zeigte, daß die Nebenproduktmenge im Zeitverlauf stetig zunahm. Eine weitere Verlängerung der Reaktionszeit wurde daher nicht untersucht.
3. Oxidation mit Chlorgas
   49,2 g 1-(4-Chlorphenyl)-pyrazolidin-3-on wurden in 300 ml Methylenchlorid gelöst. Unter Kühlung im Eisbad leitete man bei 10°C langsam 18 g Chlorgas in die Lösung ein. Die Reaktionslösung enthielt nach HPLC (Flächen-%) ca. 70 % 1-(4-Chlorphenyl)-3-hydroxy-pyrazol, 15 % Edukt und 15 % 4-Chlor-1-(4-chlorphenyl)-3-hydroxy-pyrazol.
4. Oxidation mit Brom [Chem. Heterocycl. Comp. 5, 527 (1969)]
   49,2 g 1-(4-Chlorphenyl)-pyrazolidin-3-on wurden in 300 ml Methylenchlorid gelöst. Unter Kühlung im Eisbad tropfte man bei 10°C langsam 40 g Brom ein. Die Reaktionslösung enthielt nach HPLC (Flächen-%) ca. 76 % 1-(4-Chlorphenyl)-3-hydroxy-pyrazol, 8 % Edukt und 21 % 4-Brom-1-(4-chlorphenyl)-3-hydroxy-pyrazol.

### Erfindungsgemäße Verfahrensbeispiele:

5. Herstellung von 1-(4-Chlorphenyl)-3-hydroxy-4-methylpyrazol mit Luft unter Co(II)-Katalyse
   In der Mischung aus 700 ml Wasser und 43,1 g Kaliumhydroxyid (85%) löste man 92 g 1-(4-Chlorphenyl)-4-methyl-pyrazolidin-3-on und 1,3 g Kobalt(II)acetat x 4 H₂O. Die Mischung wurde unter Rühren aufgeheizt und bei 80°C wurde über sieben Stunden Luft durchgeleitet. Nach Abkühlen wurde die Reaktionsmischung filtriert, mit Essigsäure auf pH 5,5 angesäuert, der Niederschlag abgesaugt, mit Wasser gewaschen und im Vakuum getrocknet. Man erhielt 78,9 g eines hellen Feststoffs, Fp. 214°C.
6. Herstellung von 1-(4-Chlorphenyl)-3-hydroxy-pyrazol mit Luft unter Kaliumhexacyanoferrat(III)-Katalyse
   98,3 g 1-(4-Chlorphenyl)-pyrazolidin-3-on wurden in der Mischung von 641,3 g Wasser und 33,75 g Kaliumhydroxid gelöst und mit 0,98 g Kaliumhexacyanoferrat(III) versetzt. Unter Einleiten eines kräftigen Luftstroms durch eine Kapillare wurde auf 80°C aufgeheizt und dann bei dieser Temperatur weiter oxidiert. Nach dem Abkühlen wurde mit konzentrierter Schwefelsäure auf pH 2 angesäuert. Der sich abscheidende Feststoff wurde abgesaugt, mit Wasser und Diisopropylether gewaschen und getrocknet. Es verblieben 76 g eines hellbraunen Feststoffs.
7. Herstellung von l-(4-Chlorphenyl)-3-hydroxy-pyrazol mit Luft unter Fe(III)-Katalyse
   9,06 kg 1-(4-Chlorphenyl)-pyrazolidin-3-on wurden in der Mischung aus 3,87 kg Kaliumhydroxid und 73,6 kg Wasser gelöst und mit 90 g Eisen(III)chlorid versetzt. Man erhitzte auf 80-85°C und leitete einen kräftigen Luftstrom durch. Nach ca. 3 h war die Reaktion beendet und man erhielt eine Lösung, die nach quantitativer HPLC-Analyse 8,72 Gew.-% (entsprechend 7,53 kg) an 1-(4-Chlorphenyl)-3-hydroxy-pyrazol enthielt.
8. Herstellung von 1-(4-Chlorphenyl)-3-hydroxy-pyrazol mit reinem Sauerstoff ohne Katalyse unter Druck
   Die Lösung von 9,75 g l-(4-Chlorphenyl)-pyrazolidin-3-on in 150 g Wasser wurde in einen 300 ml-Autoklaven gefüllt. Anschließend wurden 15 bar Sauerstoff aufgepreßt, auf 50°C aufgeheizt und sechs Stunden bei dieser Temperatur belassen. Man kühlte ab und stellte durch Zugabe von Essigsäure einen pH-Wert von 5 ein. Der ausgefallene Feststoff wurde abgesaugt, 30 min bei 60°C in Wasser digeriert, nochmals abgesaugt und getrocknet. Es verblieben 9,4 g des Produktes als farbloses Pulver mit einem Gehalt von 95,4 %.
9. Herstellung von 1-(4-Methylphenyl)-3-hydroxy-pyrazol mit reinem Sauerstoff ohne Katalyse
   25,8 g 1-(4-Methylphenyl)-pyrazolidin-3-on wurden in der Mischung aus 10,3 g Kaliumhydroxid und 196 g Wasser gelöst. Bei 60°C wurde Sauerstoff so eingeleitet, daß er gerade vollständig absorbiert wurde. Nach ca. 90 min wurde kein Sauerstoff mehr aufgenommen und die Reaktionsmischung wurde auf Raumtemperatur abgekühlt. Das Produkt wurde mit Essigsäure ausgefällt, abgesaugt, mit Wasser nachgewaschen und getrocknet. Es verblieben 21,6 g eines farblosen Feststoffs, Fp. 135-137°C.
10. Herstellung von 1-(3,4-Dichlorphenyl)-3-hydroxy-pyrazol mit reinem Sauerstoff ohne Katalyse
   11,8 g l-(3,4-Dichlorphenyl)-pyrazolidin-3-on wurden in der Mischung aus 5,9 g Kaliumhydroxid und 113 g Wasser gelöst. Bei 60°C wurde Sauerstoff eingeleitet und die Reaktion mittels HPLC verfolgt. Nach ca. 60 min war die Reaktion beendet, die Mischung wurde auf Raumtemperatur abgekühlt und das Produkt mit 6 g Essigsäure ausgefällt, abgesaugt, mit Wasser nachgewaschen und getrocknet. Es verblieben 7,3 g eines farblosen Feststoffs, Fp. 168-170°C.
11. Herstellung von 1-(3-Chlor-4-fluorphenyl)-3-hydroxy-pyrazol mit reinem Sauerstoff ohne Katalyse
   47,9 g 1-(3-Chlor-4-fluorphenyl)-pyrazolidin-3-on wurden in eine Lösung von 21,55 g Kaliumhydroxid in 409 g Wasser gegeben und bei 70°C durch Einleiten von Sauerstoff oxidiert. Nach ca. 40 min war die Reaktion beendet, die Mischung wurde auf Raumtemperatur abgekühlt und mit 23 g Essigsäure versetzt. Es fiel ein schmieriger Feststoff aus, der nacheinander in Wasser und Diisopropylether digeriert und abgesaugt wurde. Nach dem Trocknen verblieben 39 g Feststoff, der säulenchromatographisch an Kieselgel mit Cyclohexan gereinigt wurde. Man erhielt 21 g des Produktes, Fp. 157-159°C.
12. Herstellung von 1-(4-Chlorphenyl)-3-hydroxy-pyrazol mit reinem Sauerstoff ohne Katalyse
   850 g einer 7,4 %igen Lösung von l-(4-Chlorphenyl)-pyrazolidin-3-on in 5 %iger Kalilauge wurden auf 60°C aufgeheizt. Über eine Kapillare wurde Sauerstoff so in die Lösung eingeleitet, daß er gerade vollständig absorbiert wurde. Nach ca. 90 min war die Reaktion nach HPLC-Kontrolle beendet. Man erhielt 855 g einer Lösung, die einen Gehalt an 1-(4-Chlorphenyl)-3-hydroxy-pyrazol von 7,3 % aufwies.
13. Herstellung von 1-(4-Chlorphenyl)-3-hydroxy-pyrazol mit reinem Sauerstoff unter Co(II)-Katalyse
   900 g einer 6,9 %igen Lösung von l-(4-Chlorphenyl)-pyrazolidin-3-on in 5 %iger Kalilauge wurden mit 600 mg Cobalt(II)acetat versetzt und bei Raumtemperatur wurde über eine Kapillare Sauerstoff so in die Lösung eingeleitet, daß er gerade vollständig absorbiert wurde. Nach ca. 30 min war die Reaktion nach HPLC-Kontrolle beendet, wobei die Temperatur auf 40°C anstieg. Man erhielt 908 g einer Lösung, die einen Gehalt an 1-(4-Chlorphenyl)-3-hydroxy-pyrazol von 6,7 % aufwies.

## Patentansprüche

1. Verfahren zur Herstellung von N-substituierten 3-Hydroxypyrazolen der Formel I 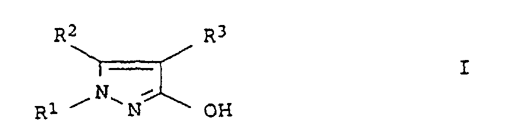 in der
R¹ für C₁-C₁₀-Alkyl, C₂-C₁₀-Alkenyl, C₂-C₁₀-Alkinyl, C₃-C₁₀-Cycloalkyl,
Aryl ausgewählt aus Phenyl und Naphthyl; Heteroaryl ausgewählt aus Furyl, Thienyl, Pyrrolyl, Isoxazolyl, Isothiazolyl, Pyrazolyl, Oxazolyl, Imidazolyl, Pyridyl, Pyridazinyl, Pyrimidinyl oder Triazinyl; und
R², R³ für Wasserstoff, Cyano, Halogen und C₁-C₁₀-Alkyl, C₂-C₁₀-Alkenyl, C₂-C₁₀-Alkinyl, C₃-C₁₀-Cycloalkyl; Aryl ausgewählt aus Phenyl und Naphthyl;
Heteroaryl ausgewählt aus Furyl, Thienyl, Pyrrolyl, Isoxazolyl, Isothiazolyl, Pyrazolyl, Oxazolyl, Imidazolyl, Pyridyl, Pyridazinyl, Pyrimidinyl oder Triazinyl; steht,
wobei die Substituenten R¹, R² und R³ unter den Reaktionsbedingungen inert sind,
durch
Oxidation eines Pyrazolidin-3-ons der Formel II 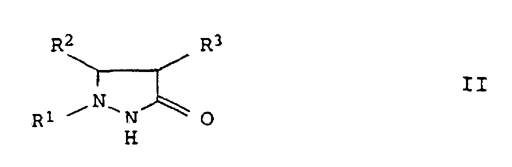 **dadurch gekennzeichnet, daß** man die Umsetzung der in Wasser löslichen Verbindungen der Formel II in Wasser in Gegenwart einer Base mit Sauerstoff als Oxidationsmittel durchführt.

2. Verfahren nach Anspruch 1, **dadurch gekennzeichnet, daß** R¹ Phenyl bedeutet, das durch die unter den Reaktionsbedingungen inerten Gruppen Halogen, Cyano, SO₃H, COOH, C₁-C₁₀-Alkyl, C₂-C₁₀-Alkenyl oder C₂-C₁₀-Alkinyl substituiert sein kann.

3. Verfahren nach Anspruch 1, **dadurch gekennzeichnet, daß** R² Wasserstoff bedeutet.

4. Verfahren nach Anspruch 1, **dadurch gekennzeichnet, daß** R³ Wasserstoff oder C₁-C₁₀-Alkyl bedeutet.

5. Verfahren nach Anspruch 1, **dadurch gekennzeichnet, daß** man eine Base mit einem pKₐ-Wert >7 verwendet.

6. Verfahren nach Anspruch 1, **dadurch gekennzeichnet, daß** man eine anorganische Base verwendet.

7. Verfahren nach Anspruch 1, **dadurch gekennzeichnet, daß** man die Oxidation von II in Gegenwart von Metallsalzen durchführt.

8. Verfahren nach Anspruch 7, **dadurch gekennzeichnet, daß** man die Metallsalze in katalytischen Mengen zusetzt.

9. Verfahren nach Anspruch 7, **dadurch gekennzeichnet, daß** man als Metallsalz ein Eisensalz verwendet.

10. Verfahren nach Anspruch 7, **dadurch gekennzeichnet, daß** man als Metallsalz ein Kupfersalz verwendet.

11. Verfahren nach Anspruch 7, **dadurch gekennzeichnet, daß** man als Metallsalz ein Kobaltsalz verwendet.

12. Verfahren nach Anspruch 1, **dadurch gekennzeichnet, daß** man die Oxidation von II mit reinem Sauerstoff als Oxidationsmittel durchführt.

13. Verfahren nach Anspruch 7, **dadurch gekennzeichnet, daß** man die Oxidation von II mit Luftsauerstoff als Oxidationsmittel durchführt.

14. Verfahren nach Anspruch 1, **dadurch gekennzeichnet, daß** man die Oxidation von II bei einem Druck von 1 bis 50 bar durchführt.

## Claims

1. A process for preparing N-substituted 3-hydroxypyrazoles of the formula I 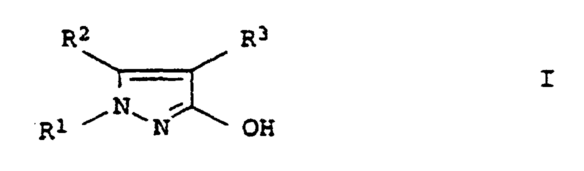 where
R¹ is C₁-C₁₀-alkyl, C₂-C₁₀-alkenyl, C₂-C₁₀-alkynyl, C₃-C₁₀-cycloalkyl,
aryl selected from the group consisting of phenyl and naphthyl,
heteroaryl selected from the group consisting of furyl, thienyl, pyrrolyl, isoxazolyl, isothiazolyl, pyrazolyl, oxazolyl, imidazolyl, pyridyl, pyridazinyl, pyrimidinyl and triazinyl; and
R², R³ are hydrogen, cyano, halogen and C₁-C₁₀-alkyl, C₂-C₁₀-alkenyl, C₂-C₁₀-alkynyl, C₃-C₁₀-cycloalkyl,
aryl are selected from the group consisting of phenyl and naphthyl,
heteroaryl selected -from the group consisting of furyl, thienyl, pyrrolyl, isoxazolyl, isothiazolyl, pyrazolyl, oxazolyl, imidazolyl, pyridyl, pyridazinyl, pyrimidinyl and triazinyl;
where the substituents R¹, R² and R³ are inert under the reaction conditions,
by
oxidizing a pyrazolidin-3-one of the formula II 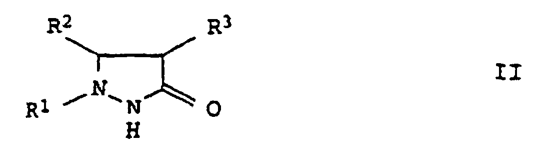 which comprises carrying out the reaction of the water-soluble compounds of the formula II in water in the presence of a base using oxygen as oxidizing agent.

2. A process as claimed in claim 1, wherein R¹ is phenyl which may be substituted by the groups halogen, cyano, SO₃H, COOH, C₁-C₁₀-alkyl, C₂-C₁₀-alkenyl or C₂-C₁₀-alkynyl which are inert under the reaction conditions.

3. A process as claimed in claim 1, wherein R₂ is hydrogen.

4. A process as claimed in claim 1, wherein R₃ is hydrogen or C₁-C₁₀-alkyl.

5. A process as claimed in claim 1, wherein a base having a pKₐ >7 is used.

6. A process as claimed in claim 1, wherein an inorganic base is used.

7. A process as claimed in claim 1, wherein the oxidation of II is carried out in the presence of metal salts.

8. A process as claimed in claim 7, wherein the metal salts are added in catalytic amounts.

9. A process as claimed in claim 7, wherein the metal salt used is an iron salt.

10. A process as claimed in claim 7, wherein the metal salt used is a copper salt.

11. A process as claimed in claim 7, wherein the metal salt used is a cobalt salt.

12. A process as claimed in claim 1, wherein II is oxidized using pure oxygen as oxidizing agent.

13. A process as claimed in claim 7, wherein II is oxidized using atmospheric oxygen as oxidizing agent.

14. A process as claimed in claim 1, wherein II is oxidized at a pressure of from 1 to 50 bar.

## Revendications

1. Procédé de préparation de 3-hydroxypyrazoles N-substitués de formule I 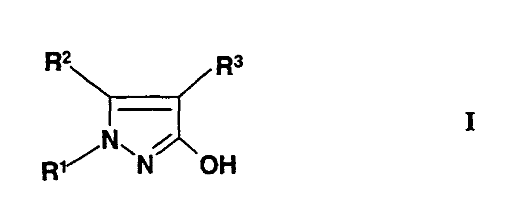 dans laquelle
R1 représente un groupe alkyle en C1 à C10, alcényle en C2 à C10, alcynyle en C2 à C10, cycloalkyle en C3 à C10;
un groupe aryle choisi parmi un groupe phényle et un groupe naphtyle;
un groupe hétéroaryle choisi parmi un groupe furyle, thiényle, pyrrolyle, isoxazolyle, isothiazolyle, pyrazolyle, oxazolyle, imidazolyle, pyridyle, pyridazinyle, pyrimidinyle, ou triazinyle; et
R2, R3 représentent un atome d'hydrogène, un groupe cyano, un atome d'halogène et un groupe alkyle en C1 à C10, alcényle en C2 à C10, alcynyle en C2 à C10, cycloalkyle en C3 à C10;
un groupe aryle choisi parmi un groupe phényle et napthyle;
un groupe hétéroaryle choisi parmi un groupe furyle, thiényle, pyrrolyle, isoxazolyle, isothiazolyle, pyrazolyle, oxazolyle, imidazolyle, pyridyle, pyridazinyle, pyrimidinyle ou triazinyle,
dans laquelle les substituants R1, R2 et R3 sont inertes dans les conditions de réaction,
par oxydation d'une pyrazolidin-3-one de formule II 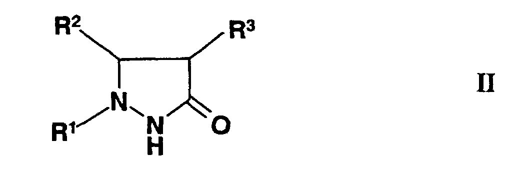 **caractérisé en ce que** l'on réalise la réaction des composés hydrosolubles de formule II dans de l'eau en présence d'une base avec l'oxygène comme agent d'oxydation.

2. Procédé selon la revendication 1, **caractérisé en ce que** R1 représente un groupe phényle qui peut être substitué par les groupes halogéno, cyano, SO3H, COOH, alkyle en C1 à C10, alcényle en C2 à C10 ou alcynyle en C2 à C10, inertes dans les conditions de réaction.

3. Procédé selon la revendication 1, **caractérisé en ce que** R2 représente un atome d'hydrogène.

4. Procédé selon la revendication 1, **caractérisé en ce que** R3 représente un atome d'hydrogène, ou un groupe alkyle en C1 à C10.

5. Procédé selon la revendication 1, **caractérisé en ce que** l'on emploie une base ayant une valeur de pKa > 7.

6. Procédé selon la revendication 1, **caractérisé en ce que** l'on emploie une base inorganique.

7. Procédé selon la revendication 1, **caractérisé en ce que** l'on réalise l'oxydation de II en présence de sels métalliques.

8. Procédé selon la revendication 7, **caractérisé en ce que** l'on ajoute les sels métalliques dans des quantités catalytiques.

9. Procédé selon la revendication 7, **caractérisé en ce que** l'on emploie un sel de fer comme sel métallique.

10. Procédé selon la revendication 7, **caractérisé en ce que** l'on emploie un sel de cuivre comme sel métallique.

11. Procédé selon la revendication 7, **caractérisé en ce que** l'on emploie un sel de cobalt comme sel métallique.

12. Procédé selon la revendication 1, **caractérisé en ce que** l'on réalise l'oxydation de II avec de l'oxygène pur comme agent d'oxydation.

13. Procédé selon la revendication 7, **caractérisé en ce que** l'on réalise l'oxydation de II avec de l'oxygène de l'air comme agent d'oxydation.

14. Procédé selon la revendication 1, **caractérisé en ce que** l'on réalise l'oxydation de II à une pression de 1 à 50 bars.
